# EUROPEAN PATENT APPLICATION

(11) **EP 3 682 759 A1**
(43) Date of publication of application: **22.07.2020**
(21) Application number: 18856495.9
(22) Date of filing: 20.08.2018
(51) Int. Cl.: A45D 20/12, A61N 5/06

(54) **HAIR DRYER**

(30) Priority: 14.09.2017 JP 2017177024
(71) Applicant: Aderans Company Limited, Shinjuku-ku Tokyo 160-8429 (JP)
(72) Inventor: MORIKAWA, Toshihide, Osaka 590-8522 (JP); TSUMURA, Yoshihiro, Tokyo 160-8429 (JP); TERAOKA, Tsuyoshi, Tokyo 160-8429 (JP)
(74) Representative: Plasseraud IP
(86) International application number: PCT/JP2018/030619
(87) International publication number: WO 2019/054131

(57) **Abstract**

To provide a hair drier capable of emitting red LED light to the scalp efficiently to improve the scalp environment, a hair drier of the present invention is arranged such that a cover body (3) provided at an air outlet (12) of a blower (14) has a facing surface (31a) that faces the head of the user in a state where the cover body (3) is covering at least a portion of the head of the user, the facing surface (31a) being provided with an LED light-emitting section (34) configured to emit LED light to the scalp of the head.

## Description

### Technical Field

The present invention relates to a hair drier.

### Background Art

It has already been known to emit red LED light to the scalp to improve the scalp environment (see, for example, Patent Literature 1).

### Citation List

### [Patent Literature]

[Patent Literature 1]
Japanese Patent Publication No. 6129392 (Registration date: April 21, 2017)

### Summary of Invention

### Technical Problem

While Patent Literature 1 discloses that emitting red LED light to the scalp is effective in improving the scalp environment, it neither discloses nor suggests how to emit red LED light to the scalp efficiently.

An aspect of the present invention has been accomplished in view of the above issue. It is an object of an aspect of the present invention to provide a hair drier configured to emit red LED light to the scalp efficiently.

### Solution to Problem

In order to attain the above object, a hair drier in accordance with an aspect of the present invention a cover body that is provided at an air outlet of a blower main body having an air inlet and the air outlet and that is configured to cover at least a portion of a head of a user, the cover body having a facing surface that faces the head of the user in a state where the cover body is covering at least the portion of the head of the user, the facing surface being provided with an LED light-emitting section configured to emit LED light to a scalp of the head.

### Advantageous Effects of Invention

An aspect of the present invention allows red LED light to be emitted to the scalp efficiently, and can thereby improve the scalp environment.

### Brief Description of Drawings

Fig. 1 is a side view of a hair drier in accordance with Embodiment 1 of the present invention.
Fig. 2 is a perspective view of the hair drier illustrated in Fig. 1.
Fig. 3 is a side cross-sectional view of the hair drier illustrated in Fig. 1, illustrating the internal structure of the hair drier.
Fig. 4 is a side cross-sectional view of a cover body of the hair drier illustrated in Fig. 1, illustrating the internal structure of the cover body.
Fig. 5 is a side cross-sectional view of (i) a portion of the cover body of the hair drier illustrated in Fig. 1 at which portion LED light-emitting sections are provided and (ii) a portion of the cover body which portion is near the above portion.
Fig. 6 is a drawing schematically illustrating an example of how the hair drier illustrated in Fig. 1 can be used.

### Description of Embodiments

### Embodiment 1

The description below deals with an embodiment of the present invention in detail.

### (Overview of hair drier)

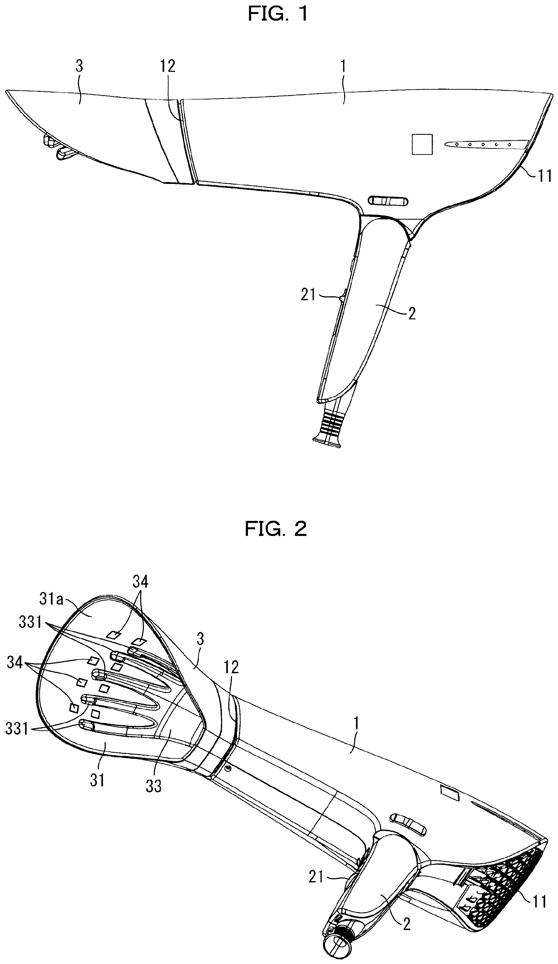

Fig. 1 is a side view of a hair drier in accordance with the present embodiment. Fig. 2 is a perspective view of the hair drier illustrated in Fig. 1.

The hair drier is, as illustrated in Fig. 1, structured to include a barrel-shaped housing 1 and a grip 2 attached to the housing 1. The housing 1 has an air inlet 11 at one end and an air outlet 12 at the other end. The housing 1 has an air flow path inside. The air inlet 11 is provided with an air inlet cover attached thereto in the shape of a wave plate. Air is sucked into the housing 1 through the air inlet cover at the air inlet 11. The air inlet 11 may alternatively be present at a side surface of the housing 1. The grip 2 is provided with an operation section 21 that the user operates to cause the hair drier to operate. The grip 2 is also provided with a power supply cord (not shown) connected thereto. The hair drier is supplied with electric power from outside through the power supply cord. The hair drier includes a cover body 3 configured to cover at least a portion of the scalp of the user to improve the scalp environment of the user. The hair drier includes at least the cover body 3 and a control section 17 configured to control light emission of each LED light-emitting section 34 (described later).

### (Internal structure of hair drier)

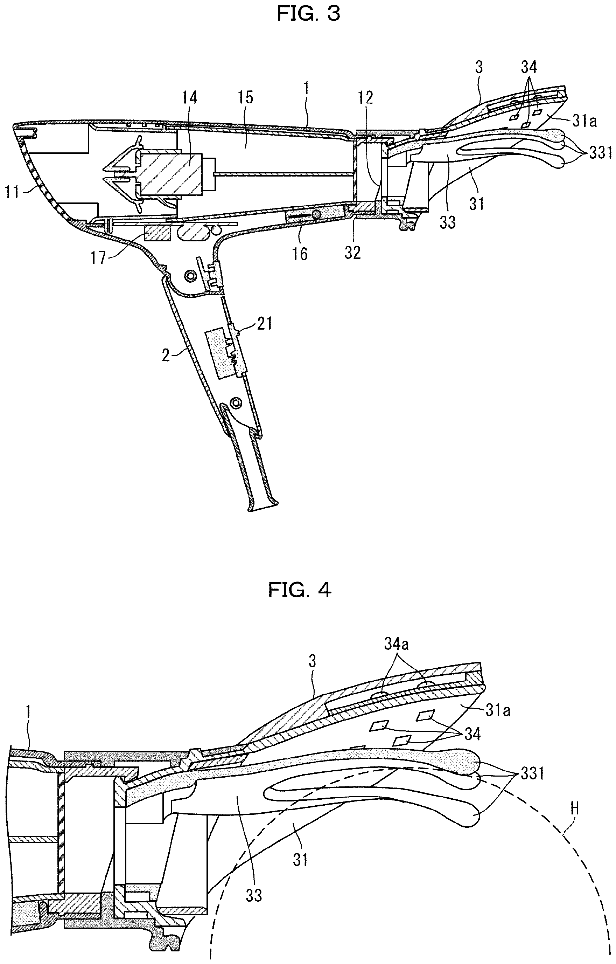

Fig. 3 is a side cross-sectional view of the drier illustrated in Fig. 1, illustrating the internal structure of the drier.

The housing 1, as illustrated in Fig. 3, contains a blower 14 configured to blow air in the direction of the barrel length of the housing 1. Fig. 3 illustrates an example of the blower 14 being an axial fan. When the blower 14 is in operation, external air is sucked through the air inlet 11 to cause an airflow through the inside of the housing 1 as an air flow path, and the airflow is then discharged through the air outlet 12. The housing 1 contains, between the blower 14 and the air outlet 12, a heater 15 configured to warm air. The heater 15 is, for example, structured to include (i) a support plate inside the housing 1 and (ii) a heating wire wound around the support plate. The heater 15 is configured to warm air inside the housing 1 so that airflow to be discharged through the air outlet 12 is warm.

The hair drier includes an ion generating unit (electrically charged particle generating section) 16 configured to generate air ions (electrically charged particles) in the atmosphere. The ion generating unit 16 is contained in the housing 1. The ion generating unit 16 includes a needle-shaped discharge electrode and a ring-shaped induction electrode around the discharge electrode. Voltage is applied between the discharge electrode and the dielectric electrode to generate corona discharge, which generates air ions around the discharge electrode. The ion generating unit 16 includes two pairs of the discharge electrode and the dielectric electrode to generate both positive air ions such as H⁺(H₂O)ₘ and negative air ions such as O₂-(H₂O)ₙ. The ion generating unit 16 generates positive and negative air ions in the air inside the housing 1. Air ions generated by the ion generating unit 16 are mixed with air inside the housing 1, so that airflow containing air ions is discharged through the air outlet 12.

The hair drier includes a control section 17 configured to control how each section is operated. The control section 17 is connected to the operation section 21, the blower 14, the heater 15, and the ion generating unit 16. The control section 17 controls the respective operations of the blower 14, the heater 15, and the ion generating unit 16 in accordance with the user's operation accepted by the operation section 21.

The control section 17 is also configured to control light emission of each LED light-emitting section 34 provided on the cover body 3. The control section 17 may be configured to keep the blower 14 and the ion generating unit 16 out of operation while the control section 17 keeps the heater 15 out of operation.

### (Structure of cover body)

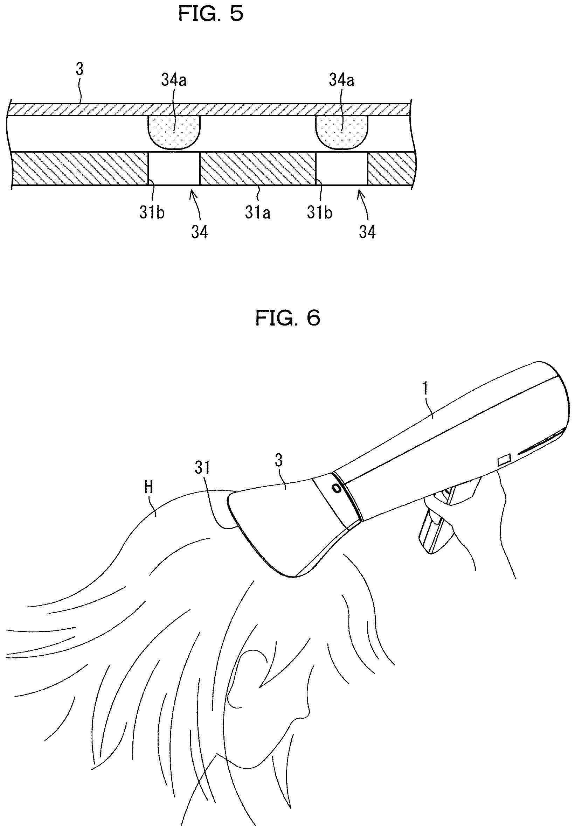

Fig. 4 is a side cross-sectional view of the cover body 3, illustrating the internal structure of the cover body 3. Fig. 5 is a side cross section of (i) a portion of the cover body 3 at which portion LED light-emitting sections 34 are provided and (ii) a portion of the cover body 3 which portion is near the above portion.

As illustrated in Fig. 4, the cover body 3 is substantially in the shape of a barrel and has an opening section 31. The cover body 3 forms an air flow path inside the opening section 31. The cover body 3 includes, inside the air flow path, a head stimulating body 33 configured to stimulate the head of the user. The head stimulating body 33 is similar in shape to a human hand. The head stimulating body 33 includes a plurality of bar-shaped bodies 331 each similar in shape to a finger. The plurality of bar-shaped bodies 331 are each oriented to extend beyond the opening section 31 so that the tip thereof protrudes from the cover body 3. The head stimulating body 33 is configured to come into contact with the head H of the user. Specifically, the bar-shaped bodies 331 of the head stimulating body 33 rake through hair of the head H so that the respective tips of the bar-shaped bodies 331 come into contact with the scalp to stimulate the scalp. The head stimulating body 33 is thus capable of stimulating the head of the user.

As illustrated in Fig. 4, the cover body 3 has a facing surface 31a facing the head H on the side of the opening section 31. The facing surface 31a is provided with a plurality of LED light-emitting sections 34 each configured to emit LED light to the scalp of the head. The LED light-emitting sections 34 are, for instance, each positioned on the facing surface 31a of the cover body 3 so that the LED light is not blocked by a bar-shaped body 331 of the head stimulating body 33 as illustrated in Fig. 2. While Fig. 2 illustrates eight LED light-emitting sections 34 as an example, the number of LED light-emitting sections 34 is not limited to any particular number.

The LED light-emitting sections 34, as illustrated in Fig. 5, each include an LED 34a inside an opening 31b in the facing surface 31a at the opening section 31 of the cover body 3. The LED 34a is a red LED configured to emit red LED light. The red LED is, for example, an LED configured to generate red light having a peak wavelength within a range of 630 nm to 660 nm. The red LED light emitted by the LED 34a travels through an opening 31b to strike the scalp of the head H with which the bar-shaped bodies 331 of the head stimulating body 33 are in contact.

### (Use example)

Fig. 6 is a drawing schematically illustrating an example of how the hair drier in accordance with Embodiment 1 can be used. The user holds the hair drier in such a manner that the opening section 31 faces the head of the user and that the cover body 3 covers a portion of the head of the head. When the user is holding the hair drier as above, the opening section 31 is in contact with or near the head H of the user. In this state, the head stimulating body 33, which is provided at the opening section 31 of the cover body 3, is in contact with the head H of the user. Specifically, the bar-shaped bodies 331 of the head stimulating body 33 rake through hair so that the respective tips of the bar-shaped bodies 331 come into contact with the scalp to stimulate the scalp. The head stimulating body 33 is thus capable of stimulating the head H of the user.

When the cover body 3 is pressed down against the head H of the user, the hair drier discharges, through the air outlet 12, unheated air containing electrically charged particles. The air then passes through the air flow path inside the cover body 3 and touches the head H of the user.

While the cover body 3 is being pressed down against the head H of the user, the LED 34a of each LED light-emitting section 34 emits red LED light to the scalp of the head H of the user.

Thus, when the cover body 3 is pressed down against the head H of the user, and the LED 34a of each LED light-emitting section 34 emits red LED light, the hair drier discharges, through the air outlet 12, unheated air containing air ions. The air then passes through the air flow path inside the cover body 3 and touches the head H of the user. The air discharged is not dissipated immediately, and remains inside the cover body 3 (which is covering a portion of the head of the user) for a certain time period. The air ions contained in the air are thus not dissipated and remain inside the cover body 3, thereby acting locally on that portion of the head of the user which is covered by the cover body 3. As the air ions act locally, hair and scalp at that portion which is covered by the cover body 3 are acted on by air ions at a density higher than conventional. As the air ions remain inside the cover body 3, hair and scalp are acted on by air ions for a time period longer than conventional.

Air containing air ions touches the head while the plurality of bar-shaped bodies 331 of the head stimulating body 33 have raked through hair. This allows air ions to come into contact with hair and scalp efficiently, so that the air ions act on the hair and scalp with a high efficiency. This in turn makes it possible to improve the environment of hair and scalp by, for example, removing static electricity and bacteria from the hair and scalp and retaining moisture on the hair and scalp. Further, air ions act on the scalp of the user while the head stimulating body 33 is stimulating the scalp. This is expected to improve the scalp environment.

The LED 34a of each LED light-emitting section 34 emits red LED light to the scalp while the plurality of bar-shaped bodies 331 of the head stimulating body 33 have raked through hair. This is expected to improve the environment of hair and scalp.

As described above, in a case where the LED 34a of each LED light-emitting section 34 of the cover body 3 emits light, the heater 15 does not warm air, and no warmed air is discharged from the air outlet 12. If air has been warmed, warmed air will continue to locally touch a portion of the head of the user, so that heat will not be dissipated easily from the surface that the air touches. The present embodiment is configured to not warm air in the case where the LED 34a of each LED light-emitting section 34 of the cover body 3 emits light. This allows unwarmed air to touch the head of the user, so that heat is dissipated easily from the surface that the air touches.

Aspects of the present invention can also be expressed as follows:
A hair drier in accordance with a first aspect of the present invention a cover body 3 that is provided at an air outlet 12 of a blower 14 main body having an air inlet 11 and the air outlet 12 and that is configured to cover at least a portion of a head H of a user, the cover body 3 having a facing surface 31a facing the head H in a state where the cover body is covering the portion of the head H of the user, the facing surface 31a being provided with an LED light-emitting section 34 configured to emit LED light to a scalp of the head H.

With the above arrangement, LED light emitted by the LED light-emitting section provided on the cover body directly strikes the scalp of the user from an extremely small distance. This means that the above arrangement allows LED light to be emitted to the scalp of the user efficiently. Irradiating the scalp of the user with LED light with a high illuminance efficiently as described above makes it possible to improve the scalp environment with LED light.

A hair drier in accordance with a second aspect of the present invention is arranged as in the first aspect and may be further arranged such that the blower 14 main body is provided with an electrically charged particle generating section (ion generating unit 16) configured to generate an electrically charged particle in air, and discharges, through the air outlet 12, air containing the electrically charged particle.

With the above arrangement, discharging air containing electrically charged particles from the air outlet of the blower main body causes the discharged air to remain inside the cover body. This allows the electrically charged particles contained in the air to act locally on that portion of the head of the user which is covered by the cover body. This can improve the scalp environment.

Thus, the hair drier arranged as above allows red LED light to be emitted to the scalp efficiently, and can thereby improve the scalp environment.

A hair drier in accordance with a third aspect of the present invention is arranged as in the first or second aspect and may be further arranged such that the LED light-emitting section 34 emits red light having a peak wavelength within a range of 630 nm to 660 nm.

With the above arrangement, the LED light-emitting section is a red LED configured to emit red LED light. This can improve the scalp environment further.

A hair drier in accordance with a fourth aspect of the present invention is arranged as any one of the first to third aspects and may be further arranged such that the cover body 3 is provided with a head stimulating body 33 configured to stimulate the head H of the user in a state where the cover body 3 is covering the head H of the user.

With the above arrangement, in a case where the head of the user is covered by the cover body, the bar-shaped bodies of the head stimulating body rake through hair and stimulate the scalp, and LED light is emitted to the scalp at the same time. This can improve the scalp environment.

The present invention is not limited to the embodiments, but can be altered by a skilled person in the art within the scope of the claims. The present invention also encompasses, in its technical scope, any embodiment derived by combining technical means disclosed in differing embodiments. Further, it is possible to form a new technical feature by combining the technical means disclosed in the respective embodiments.

### Reference Signs List

1 Housing
2 Grip
3 Cover body
11 Air inlet
12 Air outlet
14 Blower
15 Heater
16 Ion generating unit (electrically charged particle generating section)
17 Control section
21 Operation section
31 Opening section
31a Facing surface
31b Opening
33 Head stimulating body
34 LED light-emitting section
34a LED
331 Bar-shaped body

## Claims

1. A hair drier, comprising:
a cover body that is provided at an air outlet of a blower main body having an air inlet and the air outlet and that is configured to cover a portion of a head of a user,
the cover body having a facing surface that faces the head of the user in a state where the cover body is covering at least the portion of the head of the user, the facing surface being provided with an LED light-emitting section configured to emit LED light to a scalp of the head.

2. The hair drier according to claim 1, wherein
the blower main body is provided with an electrically charged particle generating section configured to generate an electrically charged particle in air, and discharges, through the air outlet, air containing the electrically charged particle.

3. The hair drier according to claim 1 or 2, wherein
the LED light-emitting section emits red light having a peak wavelength within a range of 630 nm to 660 nm.

4. The hair drier according to any one of claims 1 to 3, wherein
the cover body is provided with a head stimulating body configured to stimulate the head of the user in a state where the cover body is covering the head of the user.
